# EUROPÄISCHE PATENTSCHRIFT

(11) **EP 0 985 376 B1**
(45) Veröffentlichungstag und Bekanntmachung des Hinweises auf die Patenterteilung: **04.11.2009**
(21) Anmeldenummer: 99117242.0
(22) Anmeldetag: 02.09.1999
(51) Int. Cl.: A61B 5/15

(54) **System geeignet zur Gewinnung einer Körperflüssigkeit, Lanzettenmagazin und Verwendung eines solchen Systems**
system suitable for extraction of a bodily fluid, a lancet cartridge and a method of using said system
système adapté pour l'extraction d'un liquide Corporel, cartouche de lancettes et méthode d'utilisation correspondante

(30) Priorität: 07.09.1998 DE 19840856
(43) Veröffentlichungstag der Anmeldung: 15.03.2000
(73) Patentinhaber: Roche Diagnostics GmbH, 68305 Mannheim (DE)
(72) Erfinder: Kuhr, Hans-Jürgen, Dr., 68219 Mannheim (DE); Kintzig, Hans, 67311 Tiefenthal (DE)

(56) Entgegenhaltungen:
- EP-A- 0 565 970
- DE-B1- 2 803 345
- US-A- 5 514 152

## Beschreibung

Die Erfindung betrifft ein System geeignet zur Gewinnung einer Körperflüssigkeit, insbesondere von Blut, aus einer Körperpartie einer zu untersuchenden Person, wobei das System eine Stechhilfe, zwei oder mehrere Lanzetten und ein Lanzettenmagazin zur Bevorratung von zwei oder mehreren Lanzetten enthält. Die Erfindung betrifft weiterhin ein Lanzettenmagazin, das zum Einsatz in dem erfindungsgemäßen System geeignet ist, sowie die Verwendung des erfindungsgemäßen systems in einem Verfahren zur Entnahme einer Lanzette aus einem Lanzettenmagazin.

Die Untersuchung von Blutproben ermöglicht in der klinischen Diagnostik das frühzeitige und zuverlässige Erkennen von pathologischen Zuständen sowie die gezielte und fundierte Kontrolle von Körperzuständen. Die medizinische Blutdiagnostik setzt stets die Gewinnung einer Blutprobe des zu untersuchenden Individuums voraus. Während in Kliniken und bei niedergelassenen Ärzten oftmals durch eine Venenpunktion mehrere Milliliter Blut einer zu untersuchenden Person für die Analyse gewonnen werden, um damit eine Vielzahl von Labortests durchführen zu lassen, reichen für einzelne Analysen, die gezielt auf einen Parameter gerichtet sind, heutzutage oftmals wenige Mikroliter Blut aus. Solch geringe Blutmengen erfordern keine Venenpunktion. Vielmehr genügt es hier, zur Blutgewinnung durch die Haut z. B. in die Fingerbeere oder das Ohrläppchen der zu untersuchenden Person mit Hilfe einer steilen, scharfen Lanzette zu stoßen, um so einige wenige Mikroliter Blut für die Analyse zu gewinnen. Insbesondere eignet sich diese Methode, wenn die Analyse der Blutprobe unmittelbar nach der Blutgewinnung durchgeführt werden kann.

Vor allem im Bereich des sogenannten "Home-Monitoring", also dort, wo medizinische Laien selbst einfache Analysen des Bluts durchführen, und dort insbesondere für die regelmäßige, mehrmals täglich durchzuführende Blutgewinnung durch Diabetiker für die Kontrolle der Blutglucosekonzentration, werden Lanzetten und dazu passende Geräte (sogenannte Blutentnahmegeräte, Blutlanzettenvorrichtungen oder - wie sie im Folgenden genannt werden sollen - Stechhilfen), angeboten, die eine möglichst schmerzarme und reproduzierbare Blutgewinnung ermöglichen. Zudem soll die Verwendung von Lanzetten mit Stechhilfen die psychologische Schwelle beim Stechen des eigenen Körpers senken, was vor allem für Kinder, die an Diabetes erkrankt sind und auf regelmäßige Blutglucosetests angewiesen sind, von besonderer Bedeutung ist. Als Beispiele für Lanzetten und Stechhilfen seien die kommerziell erhältlichen Geräte und Lanzetten Glucolet^{®} der Bayer AG und Softclix^{®} der Boehringer Mannheim GmbH genannt. Solche Lanzetten und Geräte sind z. B. Gegenstand von EP-A 0 565 970, US 4,442,836 oder US 5,554,166.

Bei den derzeit verfügbaren Systemen erfolgt die Bereitstellung der Lanzetten für die Verwendung in Stechhilfen meist in loser Form. Der Benutzer entnimmt manuell vor jedem Stechvorgang eine Lanzette aus einer Verpackung, beispielsweise einer Pappschachtel oder einer Röhre, in der eine Vielzahl von Lanzetten ungeordnet, lose geschüttet enthalten sind. Anschließend wird die Stechhilfe, beispielsweise durch Abschrauben oder Abziehen einer Schutzkappe, für die Aufnahme der Lanzette vorbereitet, wobei der Lanzettenhalter der Stechhilfe freigelegt wird. Der Lanzettenhalter dient einerseits der Aufnahme der Lanzetten. Andererseits wird durch ihn die Lanzette beim eigentlichen Stechvorgang geführt. Die aus der Packung entnommene Lanzette wird manuell in den Lanzettenhalter der Stechhilfe eingeführt und dort fixiert. Dann muß die Schutzhülle, welche die Lanzettenspitze umgibt und sowohl diese als auch den Benutzer schützt, von der Lanzette manuell abgenommen werden. Anschließend wird die Stechhilfe mit ihrer Schutzkappe wieder verschlossen. Die Schutzkappe sorgt dafür, daß die Lanzette von außen nicht mehr zugänglich ist. Sie besitzt meist eine Öffnung, durch welche die Lanzettenspitze beim eigentlichen Stechvorgang austreten kann. Schließlich wird die Stechhilfe gespannt und steht für den Stechvorgang zur Gewinnung von Blut zur Verfügung.

Die Vielzahl der manuellen Bedienschritte wird vom Benutzer als nachteilig empfunden und ist vor allem bei eingeschränkter Wahrnehmung im Zustand einer Hypoglykämie problematisch. Zudem wird der Benutzer dazu verleitet, eine einmal eingelegt Lanzette mehrfach zum Stechen und Blutgewinnen zu verwenden. Dies ist zum einen aus hygienische Gründen bedenklich. Zum anderen führt die mehrmalige Benutzung der Lanzetten zu steigendem Schmerz für den Benutzer, denn da die Lanzetten als Einmalartikel konzipiert sind, werden sie schnell stumpf Zudem besteht mit den Stechhilfen und Lanzetten des Standes der Technik die Gefahr, daß

Stechhilfen mit nicht passenden Lanzetten benutzt werden oder daß die Lanzetten unsachgemäß in die Stechhilfen eingelegt werden. Weiterhin kann sich ein Benutzer bei unsachgemäße Benutzung von Lanzetten und Stechhilfen unbeabsichtigt verletzen.

Es mangelt deshalb nicht an Versuchen, die angesprochenen Nachteile zu beseitigen. Aus den US-Patentschriften US 3,030,959, US 4,794,926, US 5,035,704 und US 5,152,775 sind Stechhilfen bekannt, die mehrere Lanzetten in sich bevorraten und diese nacheinander einzeln für Stechvorgänge benutzen können. Aus DE2803345 B1, US 5,514,152 und WO 98/14125 sind miteinander verbundene Lanzetten bekannt, die gemeinsam in eine Art Stechhilfe eingeführt werden können. Die in diesen Schriften vorgeschlagenen Konzepte können die angesprochenen Probleme jedoch nur partiell lösen und erzeugen teilweise selbst neue Nachteile wie aufwendige Kostruktion von Stechhilfe und/oder Lanzetten, mangelnde Hygiene und/oder Sterilität und ungenügenden Bedienkomfort.

Aufgabe der Erfindung ist es, die Nachteile des Standes der Technik zu beseitigen. Insbesondere ist es die Aufgabe der vorliegenden Erfindung, die Anzahl der manuellen Bedienschritte beim Einlegen einer Lanzette in eine Stechhilfe zu reduzieren und somit den Bedienkomfort für den Benutzer zu erhöhen. Zudem ist es Aufgabe der Erfindung, ein verwechslungsfreies Einlegen der Lanzette in die Stechhilfe zu gewährleisten und die Sicherheit für den Benutzer im Umgang mit Stechhilfe und Lanzette, insbesondere bei hypoglykämische Zuständen, zu erhöhen.

Die Aufgabe wird durch den Gegenstand der Erfindung, wie er in den Patentansprüchen charakterisiert ist, gelöst.

Gegenstand der Erfindung ist ein System geeignet zur Gewinnung einer Körperflüssigkeit, insbesondere von Blut aus einer Körperpartie einer zu untersuchenden Person enthaltend eine Stechhilfe, die zur Aufnahme einer Lanzette geeignet ist, ein Lanzettenmagazin zur Bevorratung von zwei oder mehreren Lanzetten, das eine Transportvorrichtung für die Lanzetten aufweist und eine Öffnung besitzt, in welche die Stechhilfe zur Entnahme einer Lanzette aus dem Lanzettenmagazin eingeführt werden kann, und zwei oder mehrere Lanzetten.

Das erfindungsgemäße System ist zur Gewinnung einer Körperflüssigkeit, insbesondere von Blut, einer zu untersuchenden Person geeignet. Dabei durchstößt die von der Stechhilfe gehaltene, geführte und angetriebene Lanzette kurzzeitig und mit definierter Einstichtiefe die Haut dieser Person, wodurch eine winzige Wunde entsteht. Auf der Oberfläche der Wunde sammelt sich ein Tropfen der Körperflüssigkeit, insbesondere Blut von in aller Regel wenigen Mikroliter bis maximal 100 Mikroliter Volumen. Vorzugsweise wird die Körperflüssigkeit direkt im Anschluß an die Gewinnung für eine diagnostische Untersuchung eingesetzt. Die Probe der Körperflüssigkeit kann jedoch auch für eine spätere Untersuchung aufbewahrt werden.

Insbesondere kann mit dem erfindungsgemäßen System Kapillarblut aus einer Körperpartie, wie z. B. einer Fingerbeere oder einem Ohrläppchen, gewonnen werden. Das System kann sowohl von der zu untersuchenden Person selbst, beispielsweise einem Diabetiker, der seinen Blutglucosegehalt bestimmen möchte, als auch von einem Dritten, z. B. einem Arzt oder einer Krankenschwester zur Gewinnung von Blutproben eines Patienten, angewendet werden.

Das erfindungsgemäße System enthält eine Stechhilfe, Lanzetten und ein Lanzettenmagazin, die in ihrer Form und Funktion so aufeinander abgestimmt sind, daß ein optimales Zusammenwirken der einzelnen Systemkomponenten miteinander erreicht wird. Die einzelnen Komponenten und deren Zusammenwirken sollen im Folgenden näher erläutert werden.

### Lanzettenmagazin

Zentrale Komponente des erfindungsgemäßen Systems ist das Lanzettenmagazin. Es dient der Aufnahme, Aufbewahrung und dem Zurverfügungstellen der Lanzetten und stellt die funktionale Verbindung zwischen Lanzetten und Stechhilfe her. Zur Erfüllung dieser Zwecke besitzt das Lanzettenmagazin
- ein Gehäuse, in dem die Lanzetten aufbewahrt werden,
- eine Entnahmestelle für eine Lanzette und gegebenenfalls Mittel, die eine Lanzette in der Entnahmestelle halten,
- eine Vorrichtung, mit der die Lanzetten manuell oder automatisch in die Entnahmestelle transportiert werden können,
- eine Öffnung, in welche die Stechhilfe mit ihrer Spitze zur Entnahme einer Lanzette eingeführt werden kann und die gegebenenfalls Mittel zur Führung der Stechhilfe enthält,
- gegebenenfalls eine weitere, vorzugsweise der Öffnung für die Stechhilfe gegenüberliegende Öffnung, durch welche die Schutzhülle der Lanzettennadeln nach deren Entnahme mit Hilfe der Stechhilfe aus dem Inneren des Lanzettenmagazins entfernt werden kann, und
- gegebenenfalls eine verschließbare Öffnung, durch die Lanzetten in das Magazin gefüllt werden können.

Vorzugsweise dient das Lanzettenmagazin nicht vordringlich der Sterilität der Lanzetten. Diese wird vorzugsweise durch geeignete konstruktive Maßnahmen an der Lanzette selbst verwirklicht, beispielsweise durch Einsiegeln der Lanzettenspitze in eine abnehmbare, dichte Kunststoffschutzhülle.

Das Gehäuse des Lanzettenmagazins ist aus einem Metall, beispielsweise Aluminium, oder einem Kunststoff, z. B. Polypropylen oder Polyethylen, gefertigt. Vorzugsweise ist das Gehäuse mittels Spritzguß aus einem spritzgußfähigen Material, insbesondere Kunststoff, gefertigt. Das Gehäuse kann prinzipiell jede für die Funktion des Lanzettenmagazins geeignete Form haben. Als bevorzugt hat es sich herausgestellt, daß das Lanzettenmagazin entweder die Form eines länglichen, flachen Quaders, also ähnlich einer Zigarettenschachtel, oder die Form einer flachen quadratischen, vieleckigen oder runden Scheibe, z. B. ähnlich einer Puderdose, aufweist. Selbstverständlich können Kanten und Ecken des Gehäuses aus Design- oder Ergonomiegründen abgerundet sein. Die Dimensionen des Lanzettenmagazins werden im wesentlichen durch die Größe der Lanzetten und die Anzahl der zu bevorratenden Lanzetten bestimmt. Erfindungsgemäß können zumindest zwei Lanzetten im Magazin aufbewahrt werden. Vorzugsweise dient das Lanzettenmagazin der Bevorratung von 10 bis 200 Lanzetten. Besonders bevorzugt enthält es 20 bis 50 Lanzetten.

In einer bevorzugten Ausführungsform hat das Lanzettenmagazins die Form eines flachen, länglichen Quaders. Die kurzen, schmalen Flächen entsprechen in Länge und Breite den entsprechenden Dimensionen einer einzelnen Lanzette; die langen, schmalen Flächen entsprechen in ihrer Breite der Breite einer Lanzette und in ihrer Länge mindestens der Summe der Höhen der Lanzetten, die im Magazin untergebracht werden sollen. Die Dimensionen der großen Quaderflächen ergeben sich entsprechend. Die Lanzetten sind in dieser bevorzugten Ausführungsform im Magazin sich paarweise berührend neben- bzw. übereinander oder sich gegenüberliegend, z. B. Kopf an Kopf, gestapelt und können sowohl einzeln als auch lösbar miteinander zu einem Lanzettensatz verbunden vorliegen, beispielsweise durch Verkleben oder Verschweißen der einzelnen Lanzetten an ihren Berührungsstellen, ähnlich also wie Heftklammern miteinander zu einem Satz verbunden sind, oder durch Verbindung über dünne Kunststoffstege.

In einer alternativen, ebenfalls bevorzugten Ausführungsform hat das Lanzettenmagazin die Form einer flachen quadratischen, regelmäßig oder unregelmäßig vieleckigen oder runden Scheibe. Die Lanzetten sind in dieser Ausführungsform vorzugsweise sternförmig in einer Ebene um eine zentrale Achse angeordnet, wobei besonders bevorzugt die Lanzettenspitzen in einer gemeinsamen, zentralen Kunststoffscheibe stecken und die Lanzetten so miteinander zu einer Lanzettenscheibe verbunden sind. Auf diese Weise werden zudem die Lanzettenspitzen steril gehalten.

Unabhängig von der äußeren Form des Lanzettenmagazins - ob quaderförmig oder scheibenförmig - besitzt das erfindungsgemäße Lanzettenmagazin eine Entnahmestelle oder -position für eine Lanzette. In diese Position wird mit Hilfe einer im Magazin enthaltenen Transportvorrichtung eine Lanzette zur Entnahme durch die Stechhilfe befördert. Die Transportvorrichtung dient dazu, nach der Entnahme einer Lanzette aus dem Magazin die nächste, d. h. die der entnommenen Lanzette ursprünglich benachbarte, im Magazin enthaltene Lanzette an die Entnahmestelle des Magazins zu befördern. Dabei kann die Transportvorrichtung dieses Befördern der nächsten Lanzette zur Entnahmestelle automatisch bewerkstelligen, so daß jederzeit solange Lanzetten im Magazin enthalten sind eine Lanzette in der Entnahmeposition ist, oder aber das Befördern der nächsten Lanzette geschieht nicht automatisch, durch die Entnahme einer Lanzette ausgelöst, sondern manuell durch den Benutzer. Der Transportmechanismus kann mit einem Zählwerk gekoppelt sein, der dem Anwender anzeigt, wieviele Lanzetten aus dem Magazin bereits verbraucht bzw. wieviele Lanzetten im Magazin noch vorrätig sind.

Die Transportvorrichtung kann in einer bevorzugten Ausführungsform sämtliche im Magazin verbleibenden Lanzetten nachrücken. In einer anderen Ausführungsform ist es jedoch möglich, immer nur eine, vorzugsweise die nächstgelegene, Lanzette zur Entnahmestelle weiterzutransportieren.

In einer bevorzugten Ausführungsform werden die Lanzetten manuell mit Hilfe eines von außen zu bedienenden Schiebers in die Entnahmeposition gebracht. Der Schieber kann dabei kontinuierlich oder in diskreten Schritten, beispielsweise gerastert, mit Hilfe beispielsweise eines Bedienungsknopfes in Richtung der Entnahmeposition bewegt werden und so die im

Magazin enthaltenen Lanzetten transportieren. Die Lanzetten liegen dabei vorzugsweise mit einer Seitenfläche ganz oder teilweise auf dem im Magazin befindlichen Teil des Schiebers auf.

In einer weiteren bevorzugten Ausführungsform werden die Lanzetten mit Hilfe eines automatischen Vorschubsystems, beispielsweise im Falle des quaderförmigen Magazins über eine von Schraubenfedern getriebene, linear verschiebbare Platte oder im Falle des scheibenförmigen Magazins über eine Spiralfeder, die so auf die Lanzetten einwirkt, daß sie eine geführte, ebene Kreisbewegung ausführen, in die Entnahmeposition bewegt. Das automatische Vorschubsystem kann auch von einem Motor getrieben sein.

Das Auslösen der Transportbewegung der Lanzetten im Magazin kann im Falle des automatischen Vorschubsystems manuell, beispielsweise durch Betätigen eines Schalters, erfolgen.

Es ist jedoch auch möglich, daß die Transportbewegung automatisch durch die Entnahme einer Lanzette aus dem Magazin in Gang gesetzt wird.

Sowohl in den inneren Magazinwänden als auch bei den Lanzetten können Anschläge sowie Führungsnuten und -zapfen oder -stege vorhanden sein, die für eine präzise Positionierung der Lanzetten, insbesondere während des Transports der Lanzetten in die Entnahmeposition des Magazins, sorgen.

In einer weiteren, besonders bevorzugten Ausführungsform des erfindungsgemäßen Lanzettenmagazins werden die Lanzetten mit Hilfe einer ersten Transportvorrichtung in eine vorläufige Entnahmeposition gebracht, von der aus sie mittels eines zweiten Transportmechanismus in die endgültige Entnahmeposition bewegt werden. Die erste Transportvorrichtung kann dabei sowohl ein manuell zu bedienender Schieber als auch ein automatisches Vorschubsystem sein. Die zweite Transportvorrichtung ist in dieser besonders bevorzugten Ausführungsform ein beweglicher Teil des Lanzettenmagazins, beispielsweise ein linear bewegliches, auf einer oder mehreren Federn gelagertes Teil des Gehäuses. Durch Druck auf dieses Gehäuseteil wird dieses auf eine in der vorläufigen Entnahmeposition befindliche Lanzette zubewegt und greift diese Lanzette mit einem Greifsystem, beispielsweise über ein Widerhakensystem, welches gegebenenfalls an flexiblen Zungen angebracht ist, oder über Magnete. Beim Zurückgleiten des beweglichen Teils des Magazins wird die Lanzette in die endgültige Entnahmeposition befördert und dort über das Greifsystem gehalten, bis die Lanzette mit Hilfe einer Stechhilfe aus dem Magazin entnommen wird.

Im Bereich der Entnahmestelle findet sich eine vorzugsweise verschließbare Öffnung im Magazin, in welche die Stechhilfe zumindest mit ihrer Spitze eingeführt werden kann, um so aus dem Inneren des Lanzettenmagazins eine einzelne Lanzette zu entnehmen. Geometrie und Größe der Öffnung richten sich nach den entsprechenden Dimensionen der Stechhilfe. Vorzugsweise passen Stechhilfe und Öffnung wie Schlüssel und Schloß zueinander, so daß eine eindeutige und präzise Ausrichtung dieser beiden Systemkomponenten während des Entnahmevorgangs gewährleistet ist. Um das Einführen der Stechhilfe in die Öffnung zu erleichtern, kann die Öffnung konisch oder trichterförmig gestaltet sein, so daß sie nach außen hin größer als für die Aufnahme der Stechhilfenspitze erforderlich ist. Es ist ebenfalls möglich, daß sich die Stechhilfe im Bereich ihrer Spitze, die in das Lanzettenmagazin eingeführt wird, verjüngt, um somit das Einführen zu erleichtern.

In einer bevorzugten Ausführungsform ist im Inneren der Öffnung des Magazins ein Führungszapfen vorgesehen, der in eine entsprechende Führungsnut in der Spitze der Stechhilfe eingreift. Selbstverständlich kann der Zapfen auch auf der Stechhilfe sitzen, wobei die Führungsnut dann in der Öffnung des Magazins liegt.

Besonders bevorzugt dienen Führungszapfen und Führungsnut dazu, die Stechhilfe beim Einführen in das Magazin oder beim Entfernen aus dem Magazin - bei Bewegungen also, die im wesentlich als linear und parallel zur Längsachse der Stechhilfe zu betrachten sind - automatisch und unwillkürlich zumindest teilweise um ihre Längsachse zu drehen. Diese Drehbewegung dient dazu, mit der Stechhilfe oder einem Teil der Stechhilfe die Lanzette um ihre Längsachse zu drehen, wobei die Schutzhülle, die auf der Lanzettenspitze sitzt und die in diesem Fall nicht-drehbar im Magazin gelagert ist, abgedreht wird. Aus dem Magazin wird somit die Lanzette ohne Schutzhülle entnommen; diese verbleibt zunächst im Magazin und kann gegebenenfalls über eine weitere, vorzugsweise der Öffnung zum Einführen der Stechhilfe gegenüberliegende, Öffnung aus dem Magazin entfernt, z. B. ausgeworfen, werden.

Das Lanzettenmagazin kann als Einwegmagazin (Wegwerfmagazin) konzipiert sein oder es kann über Mittel verfügen, die ein Befüllen des Magazins mit Lanzetten erlauben. Beispielsweise kann ein Teil des Gehäuses des Lanzettenmagazins über ein Scharnier beweglich sein, und somit quasi als Tür oder Klappe dienen, durch die das Magazin mit Lanzetten bestückt werden kann. Ebenfalls möglich ist es, daß ein Teil des Magazins als Schublade ausgestaltet ist, in die Lanzetten eingelegt und in das Magazin geschoben werden können.

Das Lanzettenmagazin verfügt in einer bevorzugten Ausführungsform über Mittel, die es erlauben, den aktuellen Inhalt an Lanzetten von außen zu erkennen. Beispielsweise kann das Gehäuse des Lanzettenmagazin ganz oder teilweise transparent sein. Möglich ist jedoch auch, den Füllstand über die relative Lage eines gegebenenfalls vorhandenen Schiebers zu ermitteln, beispielsweise durch Rasterung der Schieberposition mit gleichzeitigem Vorhandensein einer Füllstandsskala.

### Lanzetten

Lanzetten, die für das erfindungsgemäße System geeignet sind, sind prinzipiell im Stand der Technik, beispielsweise in EP-A 0 565 970, beschrieben. Für bevorzugte Ausführungsformen des erfindungsgemäße Systems geeignet sind auch im Handel erhältliche Lanzetten, beispielsweise Softclix^{®} II Lancet von Boehringer Mannheim GmbH.

Erfindungsgemäß bevorzugte Lanzetten besitzen eine Nadel aus Metall, Keramik oder Kunststoff, deren eines Ende (die Spitze) spitz ausgeformt ist, beispielsweise durch einen Schleifprozeß. Der hintere, von dieser Spitze abgewandte Teil der Lanzettennadel ist in einer bevorzugten Ausführungsform üblicherweise von einem Lanzettenkörper aus Kunststoff ganz oder teilweise umschlossen. Die Herstellung erfolgt üblicherweise derart, daß die Lanzettennadel in einer Kunststoffspritzform positioniert und der Lanzettenkörper angespritzt wird. Dabei kann gleichzeitig auch eine Schutzhülle aus Kunststoffüber die Spitze der Lanzette gespritzt werden. Die Schutzhülle kann dabei vom Lanzettenkörper losgelöst vorliegen. Es ist jedoch auch möglich, daß Schutzhülle und Lanzettenkörper eine Einheit bilden, wobei in diesem Fall zwischen Schutzhülle und Lanzettenkörper eine Sollbruchstelle vorgesehen ist, so daß die Schutzhülle sauber von der Lanzette abgenommen werden kann.

Erfindungsgemäß geeignet sind jedoch auch Lanzetten, die keinen umhüllenden Körper für die Lanzettennadel aufweisen. Zur Vereinfachung soll im Folgenden der Begriff "Lanzette" für alle Varianten, d. h. Lanzettennadeln mit und ohne Lanzettenkörper, verwendet werden.

Die bevorzugte Lanzette für das erfindungsgemäße System enthält eine Metallnadel mit einer scharfen Spitze, die beim Vorgang des Stechens der zu untersuchenden Person zur Blutgewinnung durch deren Haut gestochen wird. Die Metallnadel wird vorzugsweise von einem Lanzettenkörper, d. h. einem Kunststoff- oder Metallkörper, gehalten, der die Handhabung der Nadel erleichtert.

Die für das erfindungsgemäße System geeigneten Lanzetten enthalten vorzugsweise eine teilweise mit einem Kunststoffkörper umhüllte Metallnadel, wobei der Kunststoffkörper vorzugsweise Mittel enthält, die ein Greifen und Halten der Lanzette durch eine Stechhilfe ermöglichen. Zum Beispiel kann der Kunststoffkörper ein Paar von gegenüberliegenden, V-förmigen

Aussparungen, umlaufende Einkerbungen oder trichterförmige Vertiefungen besitzen, in die komplementär gestaltete Widerhaken oder elastische Zungen des Lanzettenhalters der Stechhilfe eingreifen und die Lanzette somit festhalten können. Selbstverständlich sind jedoch auch Erhebungen auf dem Lanzettenkörper möglich, die in entsprechende Aussparungen im Lanzettenhalter der Stechhilfe eingreifen können.

Weiterhin sind in einer bevorzugten Ausführungsform am Kunststoffkörper Mittel vorhanden, die verhindern, daß die Lanzette beim Greifvorgang durch die Stechhilfe im Magazin verschoben oder gar aus dem Lanzettenmagazin geschoben werden kann. Beispielsweise können am Lanzettenkörper Zapfen oder Stege vorgesehen sein, die in entsprechende Führungsnuten im Lanzettenmagazin eingreifen und so in ihrer Relativposition zur eingeführten Stechhilfe beim Greifvorgang gehalten werden. Selbstverständlich ist es auch möglich, daß der Lanzettenkörper die Nut(en) und das Magazin einen oder mehrere entsprechende, in die Nut(en) eingreifende Stege besitzt.

Die Lanzettenspitze ist im Magazin vorzugsweise durch eine Schutzhülle, beispielsweise eine Kunststoffumhüllung, geschützt. Die Schutzhülle dient einerseits dazu, die Lanzettenspitze vor äußeren schädlichen Einwirkungen, wie z. B. Verbiegen oder Verschmutzen, was einen Verlust der Sterilität der Lanzettenspitze bedeuten würde, zu schützen, und andererseits den Benutzer, beispielsweise beim Befüllen des Magazins mit Lanzetten, vor ungewolltem Stechen durch die Lanzette zu bewahren. Die Schutzhülle kann mit dem Kunststoffkörper der Lanzette verbunden sein oder von diesem getrennt sein, wobei die verbundene Variante bevorzugt ist. Die Schutzhülle kann ein Vollkunststoff sein, der die Lanzettenspitze vollständig berührend umschließt, oder in Form eines Hohlkörpers um die Lanzettenspitze geformt sein. Jede Lanzette kann eine individuelle Schutzhülle besitzen, wie es bei den Lanzetten aus dem Stand der Technik bisher üblich ist. Es ist jedoch auch möglich, daß in einer bevorzugten Ausführungsform mehrere Lanzettenspitzen bzw. sämtliche im Lanzettenmagazin vorhandenen Lanzetten mit ihren Spitzen in einem gemeinsamen Kunststoffkörper, insbesondere einer gemeinsamen, zentralen Kunststoffscheibe, stecken, der als Schutzhülle für die Lanzettenspitzen dient.

Für die besonders bevorzugte erfindungsgemäße Variante des Lanzettenmagazins, bei der die Lanzetten in das Magazin nachgefüllt werden können, hat es sich als vorteilhaft herausgestellt, daß die einzelnen Lanzetten untereinander zu einem Lanzettensatz verbunden vorliegen. Besonders bevorzugt enthält ein solcher Lanzettensatz zwei oder mehrere Lanzetten, welche - beispielsweise im Bereich der Spitzen oder der Lanzettenkörper - lösbar miteinander verbunden sind. Dies kann einerseits auf die oben beschriebene Art geschehen, d. h. dadurch, daß mehrere Lanzettenspitzen bzw. sämtliche im Lanzettenmagazin vorhandenen Lanzetten mit ihren Spitzen in einem gemeinsamen, vorzugsweise zentral angeordneten Kunststoffkörper stecken, andererseits aber auch dadurch erreicht werden, daß die Kunststoffkörper oder - schutzhüllen individueller Lanzetten miteinander lösbar verbunden, z. B. verklebt sind. Die Verbindung kann dabei mit Hilfe von Klebebändern, wie es z. B. bei Elektronikbauteilen üblich ist, oder direkt, wie es beispielsweise für Heftklammern bekannt ist, erfolgen. Zudem ist es möglich, die Lanzettenkörper durch Kunststoffstege miteinander zu verbinden oder die Lanzetten über eine gemeinsame Halterung, beispielsweise in Form einer Schiene oder einer Gliederkette, lösbar aneinander zu koppeln.

### Stechhilfe

Stechhilfen sind in einer Vielzahl von Formen aus dem Stand der Technik, beispielsweise aus EP-A 0 565 970, bekannt und im Handel erhältlich, z. B. unter dem Namen Softclix^{®} II von Boehringer Mannheim GmbH. Unter Bezug auf den Stand der Technik, insbesondere auf EP-A 0 565 970, erübrigt es sich hier deshalb, auf die allgemeinen Merkmale und Funktionsweisen solcher Stechhilfen - oder Blutlanzettenvorrichtungen, wie sie auch genannt werden -

Erfindungsgemäße stechhilfen, sind längliche, zylindrische Stechhilfen, d. h. solche, die im wesentlichen die Form eines Füllfederhalters besitzen. Diese Stechhilfen besitzen im Inneren einen Mechanismus, der die Lanzette beim Stechvorgang geführt auf die gewünschte Einstichstelle in der Haut der zu untersuchenden Person zu- und nach dem Stechen wieder wegbewegt. Meist wird dieser Mechanismus durch eine gespannte Feder angetrieben, die manuell gespannt werden muß, weshalb für diese Ausführungsform geeignete Spannvorrichtungen vorhanden sein müssen. Zum Auslösen des Mechanismus kann auf der Außenseite der Stechhilfe ein Auslöseknopf vorhanden sein.

Stechhilfen in Form eines Füllfederhalters besitzen einen Griftkörper, welcher der bequemen Handhabung der Stechhilfe dient und in dessen Inneren meist ein Großteil des oben beschriebenen Mechanismus beherbergt wird. Zudem sind im Bereich des Griffkörpers oftmals die Bedienungsknöpfe zum Auslösen des Stoßmechanismus und gegebenenfalls zum Auswerfen gebrauchter Lanzetten aus der Stechhilfe vorhanden.

An einem Ende der Stechhilfe, welches als Spitze der Stechhilfe bezeichnet werden soll, befindet sich der Lanzettenhalter. Dieser kann unter einer abnehmbaren Schutzkappe verborgen sein. Für die Erfindung wesentlich ist, daß der Lanzettenhalter der Stechhilfe Mittel enthält, die ein Greifen einer Lanzette ermöglichen, und die somit geeignet sind, eine Lanzette aus einem Lanzettenmagazin zu entnehmen. Der Lanzettenhalter kann zu diesem Zweck in das Lanzettenmagazin über dessen Öffnung eingeführt werden, um eine Lanzette zu entnehmen. Der Lanzettenhalter stellt quasi diejenige Spitze der Stechhilfe dar, die zur Lanzettenentnahme in das Lanzettenmagazin eingeführt wird. Seine genaue Form ergibt sich einerseits aus der Form der Lanzetten, die in ihm aufgenommen und von ihm gegriffen werden sollen, und andererseits aus der Öffnung des Lanzettenmagazins, in die er eingeführt werden soll. Vorzugsweise entspricht der Lanzettenhalter der Stechhilfe demjenigen, der aus EP-A 0 565 970 bekannt ist.

Für das erfindungsgemäße System bestehend aus Stechhilfe, Lanzettenmagazin und Lanzetten hat es sich als vorteilhaft herausgestellt, daß die Stechhilfe in dem Teil, welcher in das Lanzettenmagazin zur Entnahme einer Lanzette eingeführt wird, eine Führungsnut oder einen Führungszapfen aufweist, der mit einer entsprechenden Vorrichtung in der Öffnung des Lanzettenmagazins wechselwirken kann. Dadurch kann ein verwechslungsfreies Zusammenführen von Stechhilfe und Magazin gewährleistet werden. Zudem kann durch entsprechende Formung der Nut - sei es in der Stechhilfe oder in der Öffnung des Magazins - wie weiter oben ausgeführt eine Drehung der Stechhilfe um deren Längsachse erzwungen werden, so daß sich diese beim Einführen in das Magazin oder bei dessen Herausnehmen aus dem Magazin dreht. Dabei wird ebenfalls die Lanzette im Lanzettenhalter um ihre Längsachse gedreht und somit die Schutzhülle um die Lanzettenspitze abgedreht. Auf diese Weise wird gewährleistet, daß sich die Lanzette ohne Schutzhülle in der Stechhilfe befindet. Ein manuelles Abdrehen der Schutzhülle entfällt dadurch und die Verletzungsgefahr für den Benutzer wird minimiert. Zudem wird die Anzahl der Bewegungsabläufe, die zum Einlegen einer Lanzette in die Stechhilfe erforderlich sind, minimiert und der gesamte Vorgang des Einlegens damit für den Benutzer erleichtert.

Ein weiterer Gegenstand der Erfindung ist ein Lanzettenmagazin zur Bevorratung von zwei oder mehreren Lanzetten, welches zur Verwendung in dem erfindungsgemäßen System geeignet ist. Ein solches Lanzettenmagazin ist bereits weiter oben im Zusammenhang mit dem erfindungsgemäßen System beschrieben worden.

Schließlich ist ein weiterer Gegenstand der Erfindung die verwendung des erfindungs gemäßen systems in einem Verfahren zur Entnahme einer Lanzette aus einem Lanzettenmagazin, wobei eine Lanzette, die sich im Lanzettenmagazin befindet, manuell oder automatisch mit Hilfe einer im lauzettenengram vorhandenen Transportvorrichtung in eine Entnahmeposition im Inneren des Lanzettenmagazins transportiert wird und eine Stechhilfe teilweise in eine dafür vorgesehene Öffnung des Lanzettenmagazins eingeführt wird. Beim teilweisen Einführen der Stechhilfe greift diese automatisch die Lanzette, die sich in der Entnahmeposition befindet. Die Stechhilfe wird schließlich mit der gegriffenen Lanzette aus dem Lanzettenmagazin herausgenommen.

Vorzugsweise wird entweder beim Einführen der Stechhilfe in das Lanzettenmagazin, d. h. beim Greifen der Lanzette durch die Stechhilfe, oder beim Entnehmen der Stechhilfe aus dem Lanzettenmagazin die Stechhilfe und somit auch die Lanzette verdreht, so daß die Schutzhülle aus Kunststoff, welche die Nadelspitze der Lanzette schützt, abgedreht wird. Besonders bevorzugt wird dieses Verdrehen durch entsprechende Mittel sowohl in der Stechhilfe als auch im Lanzettenmagazin, beispielsweise eine gekrümmte Führungsnut auf der einen und einen entsprechenden Führungszapfen auf der anderen Seite, automatisch bewirkt.

Die Vorteile der Erfindung können wie folgt.zusammengefaßt werden:
◆ Durch das erfindungsgemäße System aus Lanzettenmagazin, Lanzetten und Stechhilfe wird der Lanzettenwechsel für den Benutzer vereinfacht, der Zeitaufwand dafür verringert und damit die Bereitschaft erhöht, einmal benutzte Lanzetten nicht wiederzuverwenden. Dies minimiert die Infektionsgefahr und trägt zur Reduktion des Schmerzes bei der Blutgewinnung bei. Zudem ist die Handhabung des Systems bei hypoglykämischen Zuständen erleichtert.
◆ Da die Lanzetten vom Benutzer nicht mehr separat in die Hand genommen werden müssen, können die Lanzetten deutlich kleiner als bisher übliche Lanzetten gestaltet werden, wodurch sich der Materialaufwand bei der Fertigung reduziert. Zudem fällt bei der Entsorgung der Lanzetten weniger Müll an. Schließlich kann das ganze System kompakter gehalten werden, so daß dessen Mitnahme für den Benutzer erleichtert wird.
◆ Die Lanzetten werden im Lanzettenmagazin in einer eindeutigen Ausrichtung für die Stechhilfe angeboten, so daß ein Einlegen in falscher Orientierung ausgeschlossen werden kann.
◆ Durch das Schlüssel-Schloß-Prinzip von Lanzettenmagazin und Stechhilfe ist ein Verwenden von nicht geeigneten Lanzetten für die Stechhilfe ausgeschlossen.
◆ Das Lanzettenmagazin kann als wiederverwendbarer Systembestandteil jeweils mit neuen Lanzetten befüllt werden, entweder durch den Hersteller, den Vertrieb oder den Verbraucher. Die Lanzetten selbst können deshalb platz- und müllsparend mit minimaler Verpackung in den Handel gebracht werden.

Die Erfindung wird durch die nachfolgenden Zeichnungen näher erläutert.
Figur 1 zeigt schematisch eine Seitenansicht einer bevorzugten Ausführungsform eines erfindungsgemäßen Lanzettenmagazins, bei dem eine Seitenwand entfernt wurde, um so einen Einblick in das Magazin und auf die darin enthaltenen Lanzetten zu ermöglichen.
Figur 2 zeigt schematisch eine Aufsicht auf eine bevorzugte Ausführungsform einer Lanzette, die in einem Lanzettenmagazin gemäß Figur 1 untergebracht werden kann.
Figur 3 zeigt schematisch anhand von sieben Teilfiguren (A bis G), wie mit einer Stechhilfe Lanzetten aus einem Lanzettenmagazin gemäß Figur 1 entnommen werden können.

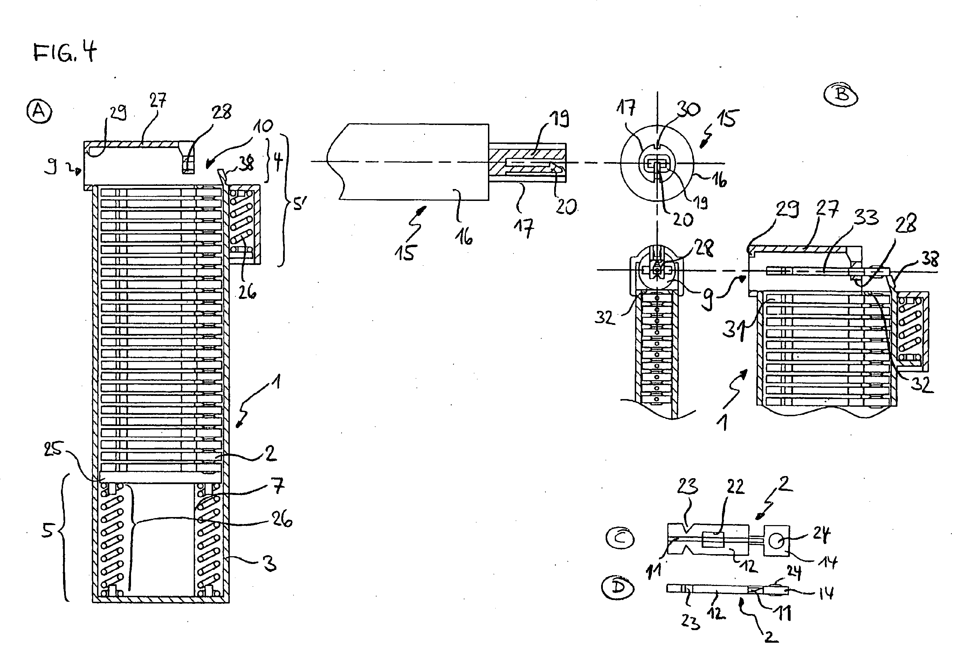

Figur 4 zeigt schematisch eine Seitenansicht einer weiteren bevorzugten Ausführungsform eines erfindungsgemäßen Lanzettenmagazins, bei dem eine Seitenwand entfernt wurde, um so einen Einblick in das Magazin und auf die darin enthaltenen Lanzetten zu ermöglichen (Teilfigur A), schematische Seiten- und Frontansichten derjenigen Teile eines bevorzugten Lanzettenspenders und einer entsprechenden Stechhilfe (Teilfigur B), die zur Lanzettenentnahme miteinander wechselwirken, sowie eine Aufsicht (Teilfigur C) und eine Seitenansicht (Teilfigur D) einer bevorzugte Ausführungsform einer Lanzette, die in dem Lanzettenmagazin gemäß Figur 4 A untergebracht werden kann.
Figur 5 zeigt schematisch anhand von zehn Teilfiguren (A bis K), wie mit einer Stechhilfe Lanzetten aus einem Lanzettenmagazin gemäß Figur 4 entnommen werden können.
Figur 6 zeigt schematisch anhand von vier Teilfiguren (A bis D) eine weitere bevorzugte Ausführungsform eines Lanzettenmagazins in Aufsicht (A) und von vorne (B), sowie einen für dieses Lanzettenmagazin geeigneten Satz von lösbar miteinander verbundenen Lanzetten, ebenfalls in Aufsicht (C) und von vorne (D). Die Ansichten von vorne (B, D) sind dabei teilweise angeschnitten, um konstruktive Details zu verdeutlichen.
Figur 7 zeigt schematisch anhand von sechs Teilfiguren (A bis F), wie mit einer Stechhilfe Lanzetten aus einem Lanzettenmagazin gemäß Figur 6 entnommen werden können.

Die Ziffern in den Figuren bedeuten:
- 1: Lanzettenmagazin
- 2: Lanzette
- 3: Gehäuse
- 4: Entnahmestelle
- 5,5': Transportvorrichtung
- 6: Bedienungsknopf für Transportvorrichtung 5
- 7: Führungsnut für Transportvorrichtung 5
- 8: Anschlag
- 9: Öffnung zum Einführen der Stechhilfe 15
- 10: Öffnung zum Auswerfen der Schutzhülle 14
- 11: Metallnadel
- 12: Lanzettenkörper
- 13: Zapfen
- 14: Schutzhülle
- 5: Stechhilfe
- 16: Griffkörper
- 17: Spitze
- 18: Schutzkappe
- 19: Lanzettenhalter
- 20: flexible Haltezunge mit Widerhaken
- 21: Auswerfer
- 22: trichterförmige Kerbe im Lanzettenkörper 12
- 23: V-förmige Aussparung im Lanzettenkörper 12
- 24: Erhebung auf Schutzhülle 14
- 25: Platte
- 26: Schraubenfeder
- 27: bewegliches Gehäuseteil
- 28: Greifersystem
- 29: Führungszapfen
- 30: Führungsnut
- 31: Lanzette 2 in vorläufiger Entnahmeposition
- 32: Haltevorrichtung
- 33: Lanzette 2 in endgültiger Entnahmeposition
- 34: Sichtfenster
- 35: Lanzettensatz
- 36: Kunststoffscheibe
- 37: Einführungsstutzen
- 38: flexible Zunge

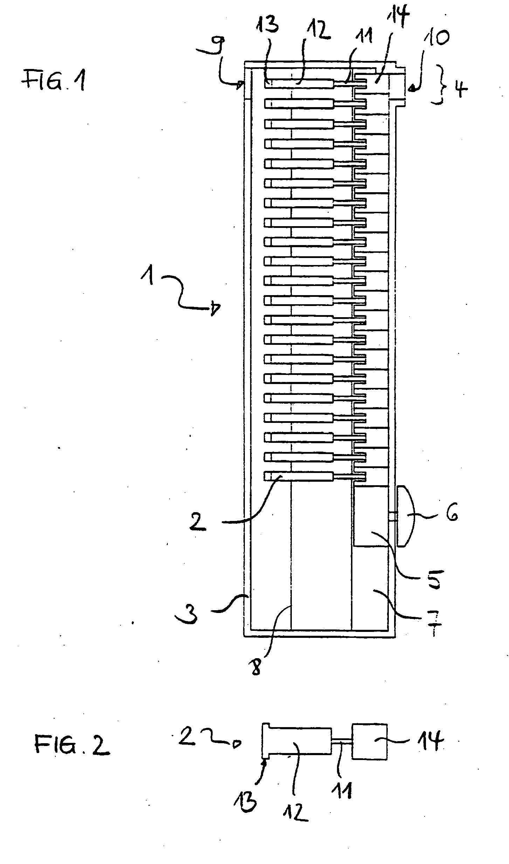

In Figur 1 ist schematisch die Seitenansicht einer besonders bevorzugten Ausführungsform eines Lanzettenmagazins (1) abgebildet, bei dem eine Seitenwand des Gehäuses (3) entfernt wurde, um so einen Einblick in das Lanzettenmagazin (1) und auf die darin enthaltenen Lanzetten (2) sowie weitere funktionale Bestandteile zu ermöglichen. Das Lanzettenmagazin (1) hat im wesentlichen die Form eines länglichen, flachen Quaders, in dem die Lanzetten (2) linear nebeneinander, sich jeweils paarweise berührend angeordnet sind. Die Lanzetten können mit Hilfe einer Transportvorrichtung (5) manuell zu Entnahmestelle (4) geschoben werden, wozu auf der Außenseite einer der langen, schmalen Seiten des Lanzettenmagazins (1) ein Bedienungsknopf (6) für die Transportvorrichtung (5) vorgesehen ist. Das Verschieben der Transportvorrichtung (5) mit Hilfe des Bedienungsknopfes (6) kann kontinuierlich oder diskontinuierlich, beispielsweise gerastert, geschehen. Die Position des Bedienungsknopfes (6) erlaubt es, den aktuellen Füllstand des Magazins (1) zu erkennen.

Innerhalb des Lanzettenmagazins (1) ist eine Führungsnut (7) enthalten, in der die Transportvorrichtung (5) und die Schutzhüllen (14) der Lanzetten (2) geführt werden. Die Schutzhüllen (14), die Transportvorrichtung (5) und die Führungsnut (7) sind in Größe und Form aneinander entsprechend angepaßt.

Ebenfalls im Inneren des Gehäuses (3) des Lanzettenmagazins (1) befindet sich ein Anschlag (8), der in Verbindung mit dem Zapfen (13) des Lanzettenkörpers (12) dafür sorgt, daß eine Lanzette (2), die sich an der Entnahmestelle (4) befindet, beim Einführen einer Stechhilfe durch die Öffnung (9) nicht aus der Öffnung (10) des Lanzettenmagazins (1) hinausgeschoben werden kann.

Die Führungsnut (7) und der Anschlag (8) können ebenfalls in der zur Innenseite des Lanzettenmagazins (1) gewandten Seitenfläche des Gehäuses (3) enthalten sein, die in Figur 1 entfernt wurde, um einen Einblick in das Lanzettenmagazin zu ermöglichen.

In Figur 2 ist eine Lanzette (2) dargestellt, die in einem Lanzettenmagazin (1) gemäß Figur 1 untergebracht werden kann. Während die Lanzette (2) in Figur 1 in einer Seitenansicht schematisch dargestellt ist, findet sich in Figur 2 eine schematische Aufsicht auf eine Lanzette (2). Die Lanzette (2) besteht im wesentlichen aus einer Metallnadel (11) mit einer Spitze, die durch eine Schutzhülle (14) ummantelt ist. Diese Schutzhülle (14) muß vor Benutzung der Lanzette (2) zur Gewinnung von Blut von der Lanzette (2) abgenommen werden. Die Lanzette (2) enthält weiterhin auf derjenigen Seite, die der Schutzhülle (14) gegenüber liegt, einen Lanzettenkörper (12), der auf beiden Seiten einen Zapfen (13) enthält. Dieser sorgt zusammen mit dem Anschlag (8) im Gehäuse (3) des Lanzettenmagazins (1) dafür, daß eine an der Entnahmestelle (4) befindliche Lanzette (2) beim Einführen einer Stechhilfe nicht aus dem Lanzettenmagazin (1) geschoben werden kann. Vorzugsweise sind der Lanzettenkörper (12) und die Schutzhülle (14) aus einem spritzgußfähigen Kunststoff gefertigt.

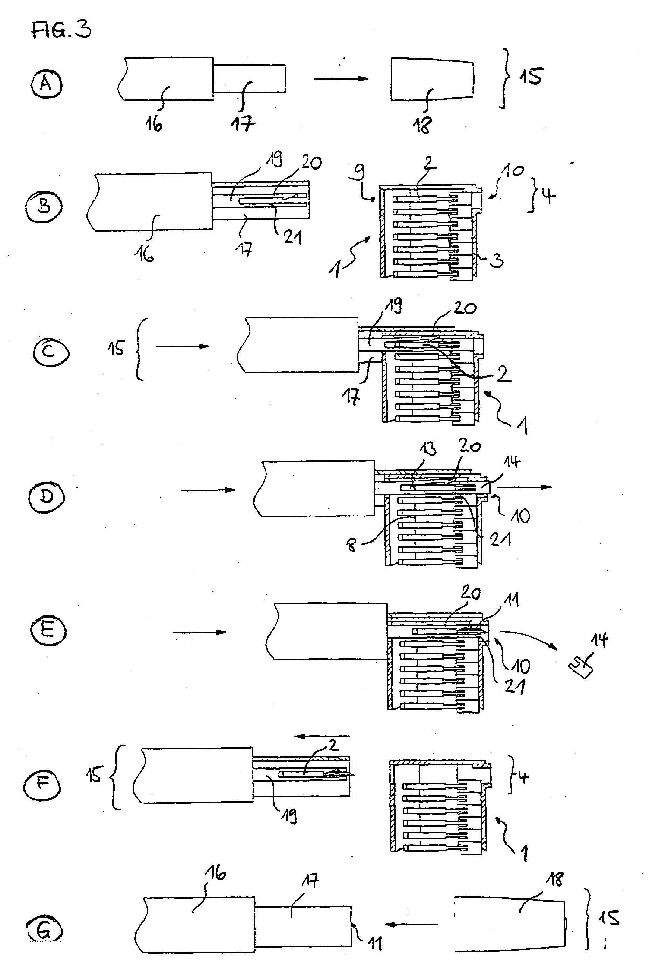

Figur 3 zeigt schematisch anhand von 7 Teilfiguren (A bis G), wie mit Hilfe einer Stechhilfe (15) eine Lanzette (2), die sich an der Entnahmestelle (4) eines Lanzettenmagazins (1) befindet, aus dem Lanzettenmagazin (1) entnommen wird. Die Stechhilfe (15) hat im wesentlichen die Form eines Füllfederhalters. Sie enthält als wesentliche Bestandteile einen Griffkörper (16), eine Spitze (17) und eine Schutzkappe (18). Im Griffkörper (16) sind die für die Stechbewegung erforderlichen, beweglichen mechanischen Bauteile der Stechhilfe (15) untergebracht. Diese entsprechen weitestgehend dem Stand der Technik, beispielsweise EP A-0 565 970, und sollen hier nicht näher erläutert werden. In der Spitze (17), die nach Abziehen oder Abschrauben der Schutzkappe (18) von der Stechhilfe (15) offenliegt, ist der Lanzettenhalter (19) untergebracht. Wesentlich am Lanzettenhalter (19) der vorliegenden, besonders bevorzugten Ausführungsform einer Stechhilfe (15) sind die flexible Haltezunge (20) mit einem Widerhaken und der Auswerfer (21), da diese Bestandteile des Lanzettenhalters (19) direkt mit der Lanzette (2) im Lanzettenmagazin (1) wechselwirken.

Nachdem die Schutzkappe (18) von der Stechhilfe (15) entfernt ist, liegt die Spitze (17) der Stechhilfe frei (Figur 3 A). Die Stechhilfe (15) wird mit ihrer Spitze (17) voran auf das Lanzettenmagazin (1) zubewegt, bei dem sich an der Entnahmestelle (4) eine Lanzette (2) befindet (Figur 3 B). Die Spitze (17) der Stechhilfe (15) besitzt eine Aussparung, die es ermöglicht, daß die Spitze (17) über das Lanzettenmagazin (1) geschoben werden kann. Dabei dringt der Lanzettenhalter (19) durch die Öffnung (9) in das Lanzettenmagazin ein. Die flexible Haltezunge (20) gleitet dabei über die an der Entnahmestelle (4) befindliche Lanzette (2), wobei die Haltezunge (20) leicht nach oben verbogen wird (Figur 3 C). Beim weiteren Einführen des Lanzettenhalters (19) in das Lanzettenmagazin (1) wird die Lanzette durch den Auswerfer (20 a) in Richtung der Öffnung (10) verschoben, bis der Zapfen (13) des Lanzettenkörpers (12) am Anschlag (8) anliegt und somit ein weiteres Verschieben der Lanzette (2) nicht mehr möglich ist (Figur 3 D). Beim weiteren Einschieben des Lanzettenhalters (19) in das Lanzettenmagazin (1) schiebt der Auswerfer (20 a) nicht mehr die gesamte Lanzette (2) sondern nur noch die Schutzhülle (14) in Richtung der Öffnung (10). Beim vollständigen Einführen des Lanzettenhalters (19) in das Lanzettenmagazin (1) wird die Schutzhülle (14) durch den Auswerfer (20 a) von der Lanzettenspitze getrennt und fällt aus der Öffnung (10). Gleichzeitig greift die flexible Haltezunge (20) mit ihrem Widerhaken vor den Lanzettenkörper (12) und hält die Lanzette (2) somit im Lanzettenhalter (19) (Figur 3 E). Beim Entnehmen der Stechhilfe (15) aus dem Lanzettenmagazin (1) wird die Lanzette (2), die sich im Lanzettenhalter (19) befindet, ebenfalls aus dem Magazin (1) entnommen. An der Entnahmestelle (4) ist so Platz für eine nachzurückende Lanzette (2) geworden, die manuell mit Hilfe der Transportvorrichtung (5) zur Entnahmestelle (4) geschoben werden kann (Figur 3 F). Nach dem Entnahmevorgang der Lanzette (2) aus dem Lanzettenmagazin (1) muß die Stechhilfe (15) mit der Schutzkappe (18) verschlossen werden. Dabei kann die Stechhilfe (15) durch Drehen der Schutzkappe (18) automatisch gespannt werden. Sie steht somit zur Blutgewinnung zur Verfügung.

In Figur 4 A ist eine weitere, besonders bevorzugte Ausführungsform des erfindungsgemäßen Lanzettenmagazins (1) in einer Seitenansicht abgebildet, in der das Lanzettenmagazin (1) teilweise angeschnitten ist. Das Lanzettenmagazin (1) hat im wesentlichen die Form eines länglichen flachen Quaders. Ähnlich wie bei der Ausführungsform der Figur 1 sind auch hier die Lanzetten (2) im wesentlich linear nebeneinander, sich paarweise berührend, angeordnet. Die Lanzetten (2) werden über eine Transportvorrichtung (5), die im wesentlichen aus einer Platte (25) und zwei diese Platte (25) antreibende Schraubenfedern (26) besteht, automatisch in Richtung der Entnahmestelle (4) transportiert. Im Bereich der Entnahmestelle (4) ist eine zweite Transportvorrichtung (5') vorgesehen. Diese besteht im wesentlichen aus einem beweglichen Gehäuseteil (27), welches durch eine Schraubenfeder (26) in der abgebildeten Ruheposition gehalten wird. Im Bereich der Transportvorrichtung (5') befindet sich auch die Öffnung (9) zum Einführen der Stechhilfe sowie die Öffnung (10) zum Auswerfen der Schutzhülle (14) der Lanzetten (2), wozu auch die flexible Zunge (38) dient, die beweglich am Gehäuse (3) des Lanzettenmagazins (1) befestigt ist. Ebenfalls Bestandteil der Transportvorrichtung (5') ist ein Greifersystem (28), welches die oberste, das heißt, der Entnahmestelle (4) nächstgelegene Lanzette (2) beim Bedienen der Transportvorrichung (5') greift.

In Figur 4 B sind Detailzeichnungen schematischer Ansichten der Stechhilfe (15) in Seiten- und Frontalansicht sowie desjenigen Teils des Lanzettenmagazins (1) abgebildet, in welchen die Stechhilfe (15) zur Entnahme einer Lanzette (2) eingeführt werden kann. Die in Figur 4 B links oben abgebildete Stechhilfe (15) ist wie die Stechhilfe (15) aus Figur 3 A nur mit dem Teil abgebildet, der für die Erfindung relevant ist. Auch hier entsprechen die übrigen funktionalen Bestandteile dem Stand der Technik. Die Stechhilfe (15) enthält einen Griffkörper (16) und eine Spitze (17), in der ein Lanzettenhalter (19) untergebracht ist, welcher wiederum eine flexible Haltezunge (20) aufweist. In Figur 4 B sind diese Bestandteile der Stechhilfe (15) teilweise angeschnitten in einer Seitenansicht zu sehen. Rechts neben der Seitenansicht in Figur 4 B ist eine Aufsicht von vorne auf die Stechhilfe (15) zu sehen. Die Aufsicht verdeutlicht die relative Lage der bereits genannten Bauteile zueinander. Zudem ist die Führungsnut (30) zu sehen, die in der Stechhilfenspitze (17) vorgesehen ist.

Unterhalb dieser Frontalansicht der Stechhilfe (15) in Figur 4 B ist eine frontale Detailansicht der Öffnung (9) des Lanzettenmagazins (1) zu sehen. Rechts daneben findet sich eine teilweise angeschnittene Seitenansicht des Lanzettenmagazins (1). In der Frontalansicht auf die Öffnung (9) des Lanzettenmagazins (1) sind insbesondere das Greifersystem (28) und der Führungszapfen (29) zu erkennen. Das Greifersystem (28) besteht aus zwei beweglichen, mit Widerhaken versehenen Zungen, die beim Niederdrücken des beweglichen Gehäuseteils (27) eine durch eine Haltevorrichtung (32), die seitlich als halbkugelförmige Erhebung aus der Innenfläche der Gehäusewand des Lanzettenmagazin (1) herausragt, in der vorläufigen Entnahmeposition (31) gehaltene Lanzette (2) umschließen und festhalten, so daß beim Entspannen der Schraubenfeder (26) und dem dadurch bewirkten Zurückgleiten des beweglichen Gehäuseteils (27) in die Ausgangsstellung eine Lanzette (2) in die endgültige Entnahmeposition (33) mitgenommen wird. Dabei wird die flexible Zunge (38) nach außen gedrückt, woraufhin sie sich wieder in ihre leicht nach innen geneigte Ruheposition zurückbewegt. Der Führungszapfen (29) bewegt sich beim Einführen der Stechhilfe (15) in die Öffnung (9) in der Führungsnut (30) in der Spitze (17) der Stechhilfe (15). Dadurch wird zum einen gewährleistet, daß die Stechhilfe (15) in der richtigen Orientierung in die Öffnung (9) eingeführt wird. Zum anderen kann durch geeignete Geometrie der Führungsnut (30) erreicht werden, daß sich die Stechhilfe (15) beim Einführen in das Lanzettenmagazin (1) über die Öffnung (9) um ihre Längsachse dreht. Auf diese Weise wird auch die Lanzette (2), die sich in der Entnahmeposition (33) befindet, um ihre Längsachse gedreht. Dabei kann die Schutzhülle (14) der Lanzette (2), wie sie in Figur 4 C und D abgebildet ist, vom Rest der Lanzette (2) abgedreht werden und somit die Metallnadel (11) bzw. deren Spitze freigeben.

Die in Figur 4 C und D dargestellte, besonders bevorzugte Ausführungsform der Lanzette (2) besteht im wesentlichen aus einer Metallnadel (11), die in diesem Fall weitgehend vollständig von einem Kunststoffkörper (12) umgeben ist. Die Schutzhülle (14) ist dabei Bestandteil des Kunststoffkörpes (12), wobei an der Verbindungsstelle zwischen Schutzhülle (14) und dem restlichen Kunststoffkörper (12) eine Sollbruchstelle vorgesehen ist, die bei einer Drehung der Schutzhülle (14) relativ zum Kunststoffkörper (12) ein Abtrennen der Schutzhülle (14) erlaubt. Auf beiden Seiten der Schutzhülle (14) befindet sich je eine Erhebung (24), die dem lösbaren Verbinden mehrerer Lanzetten (2) zu einem Lanzettensatz dienen. In der vorliegenden, bevorzugten Ausführungsform sind die Lanzetten (2) - ähnlich wie Heftklammern - über Kleberpunkte auf den Erhebungen (24) miteinander zu einem Satz von Lanzetten verbunden.

Im Lanzettenkörper (12) sind Mittel (22, 23) vorgesehen, in die Teile des Lanzettenhalters (19) eingreifen können, um die Lanzette (2) fest im Lanzettenhalter (19) zu halten. Der Lanzettenkörper (12) in der abgebildeten, besonders bevorzugten Ausführungsform der Lanzette (2) enthält zum einen eine trichterförmige Kerbe (22) und ein Paar sich gegenüberliegende V-förmige Aussparungen (23). In die trichterförmige Kerbe (22) kann der Widerhaken der beweglichen Zunge (20) des Lanzettenhalters (19) der Stechhilfe (15) eingreifen und die Lanzette (2) so festhalten. Die V-förmigen Aussparungen (23) können zusammen mit alternativen, hier nicht abgebildeten Ausführungsformen des Lanzettenhalters (19) einer Stechhilfe (15) benutzt werden.

Das Lanzettenmagazin (1) der in Figur 4 gezeigten bevorzugten Ausführungsform, kann vom Benutzer geöffnet werden, um jeweils einen neuen Lanzettensatz einzulegen. Das Gehäuse des Lanzettenmagazins (1) kann durch Wegklappen der Transportvorrichtung (5') geöffnet werden. Die Lanzetten (2) werden einzeln oder miteinander lösbar verbunden als Lanzettensatz in das Magazin (1) geschoben. Dabei wird die Transportvorrichtung (5) durch Zusammendrücken der Schraubenfedern (26) wieder gespannt. Schließlich wird die Transportvorrichtung (5') zurückgeklappt. Das Magazin (1) ist somit gebrauchsfertig.

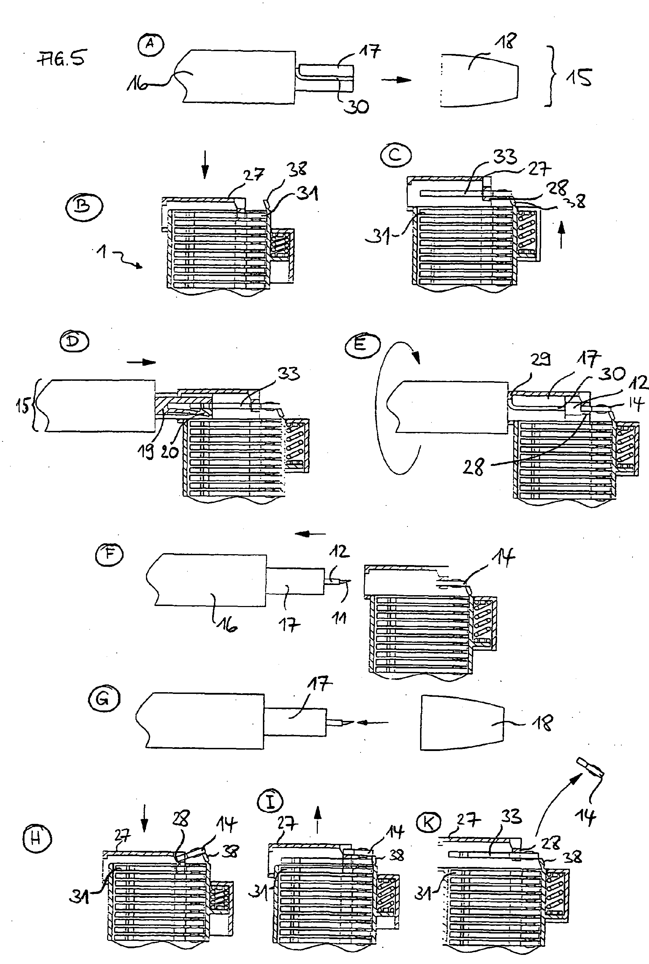

In Figur 5 ist - analog zu Figur 3 - anhand von zehn Teilfiguren (A bis K) dargestellt, wie mit Hilfe einer Stechhilfe (15) eine Lanzette (2) aus einem Lanzettenmagazin (1), welches der besonders bevorzugten Ausführungsform aus Figur 4 A entspricht, entnommen werden kann:
Vor der Entnahme der Lanzette muß die Schutzkappe (18) von der Stechhilfe (15) abgezogen bzw. abgeschraubt werden, so daß die Spitze (17) der Stechhilfe offenzuliegen kommt (Figur 5 A). Durch Drücken auf das bewegliche Gehäuseteil (27) des Lanzettenmagazins (1) wird die Lanzette, die sich in der vorläufigen Entnahmeposition (31) befindet und dort durch die Haltevorrichtung (32) gehalten wird, gegriffen (Figur 5 B) und beim Zurückgleiten des beweglichen Gehäuseteils (27) in seine Ursprungsposition unter nach außen Biegen der beweglichen Zunge (38) in die endgültige Entnahmeposition (33) bewegt, wo sie vom Greifersystem (28) gehalten wird. Die übrigen, sich im Lanzettenmagazin (1) befindlichen Lanzetten werden durch die automatische Transportvorrichtung (5) (vgl. Figur 4 A) nachgeschoben, so daß sich wieder eine Lanzette in der vorläufigen Entnahmeposition (31) befindet (Figur 5 C). Beim Einführen der Stechhilfe (15) in die Öffnung (9) des Lanzettenmagazins (1) greift der Lanzettenhalter (19) die sich in der endgültigen Entnahmeposition (33) befindliche Lanzette (2) (Figur 5 D). Durch das Zusammenwirken von Führungsnut (30) in der Stechhilfenspitze (17) und Führungszapfen (29) in der Öffnung (9) des Gehäuses (3) des Lanzettenmagazins (1) wird beim vollständigen Einführen der Spitze (17) der Stechhilfe (15) diese um ihre Längsachse gedreht. Da sich die Lanzette (2) bei der Drehung bereits weitgehend im Lanzettenhalter (19) befindet, wird auch die Lanzette (2) um ihre Längsachse gedreht. Die Schutzhülle (14) ist von dieser Drehung jedoch ausgenommen, da sie durch das Greifersystem (28) und die flexible Zunge (38) in ihrer Position fixiert ist und somit die Drehbewegung nicht ausführen kann. Der Lanzettenkörper (12) wird gegen die Schutzhülle (14) verdreht, wodurch die Sollbruchstelle zwischen Lanzettenkörper (12) und Schutzhülle (14) bricht (Figur 5 E). Beim Herausziehen der Stechhilfe (15) aus dem Lanzettenmagazin (1) wird die gegriffene Lanzette (2) mit herausgezogen. Die Schutzhülle (14) wird dabei vom Rest der Lanzette (2) getrennt und verbleibt zunächst im Greifersystem (28) (Figur 5 F). Die Spitze der Metallnadel (11) und Teile des Lanzettenkörpers (12) ragen bei dieser besonders bevorzugten Ausführungsform aus der Spitze (17) der Stechhilfe (15) heraus. Durch das Wiederaufsetzen der Schutzkappe (18) wird die freiliegende Spitze der Metallnadel (11) bedeckt (Figur 5 G). Gegebenenfalls kann gleichzeitig beispielsweise durch ein Verdrehen der Schutzkappe (18) die Stechhilfe (15) gespannt werden.

Die im Greifersystem (28) verbliebene Schutzhülle (14) der Lanzette (2) wird beim erneuten Niederdrücken des beweglichen Gehäuseteils (27) zur Entnahme einer weiteren Lanzette (2) aus der vorläufigen Entnahmeposition (31) durch die bewegliche Zunge (38) aus dem Greifersystem (28) herausgeschoben (Figur 5 H) und liegt zunächst lose auf der nächsten zur endgültigen Entnahmeposition zu transportierenden Lanzette (2) (Figur 5 I), von wo aus sie leicht entfernt werden kann (Figur 5 K).

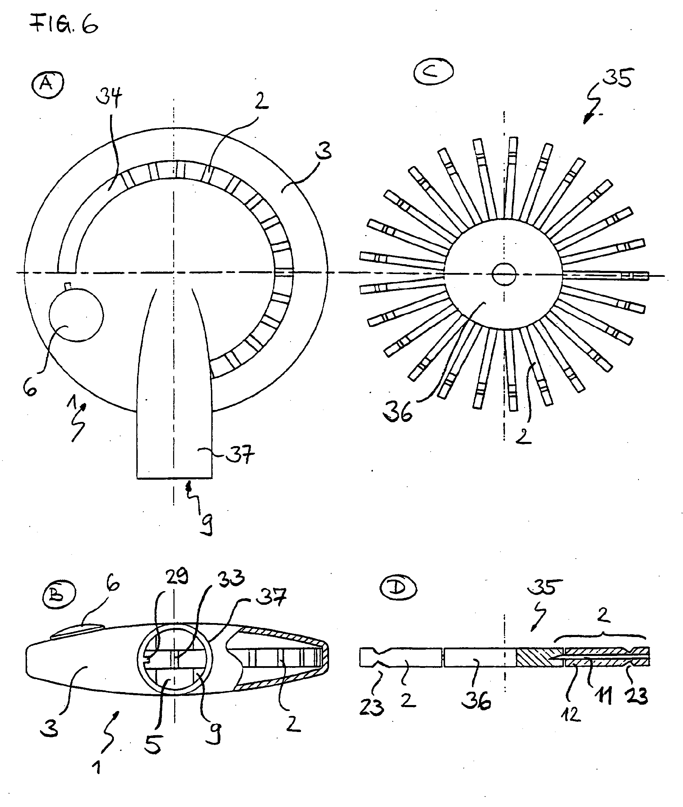

In Figur 6 ist eine weitere besonders bevorzugte Ausführungsform des erfindungsgemäßen Lanzettenmagazins (1) (A, B) sowie eines hierfür geeigneten Lanzettensatzes (35) (C, D) abgebildet. Das Lanzettenmagazin (1) dieser bevorzugten Ausführungsform hat im wesentlichen die Form einer flachen, runden Scheibe, an die ein Einführstutzen (37) angebracht ist, durch den über die Öffnung (9) eine Stechhilfe zur Entnahme von Lanzetten (2) in das Lanzettenmagazin (1) eingeführt werden kann. Auf einer der kreisförmigen Seiten des Lanzettenmagazins (1) ist ein transparentes Sichtfenster (34) vorgesehen, das es erlaubt, den aktuellen Füllstand des Lanzettenmagazins (1) mit Lanzetten (2) zu kontrollieren. Das Sichtfenster (34) kann aus einem mit dem Gehäuse (3) verbundenen transparenten Kunststoff bestehen. Es ist jedoch auch möglich, daß das gesamte Gehäuse (3) aus einem transparenten Kunststoff gefertigt ist. Alternativ dazu kann das Sichtfenster (34) lediglich als offengelassene Aussparung des Gehäuses (3) ausgestaltet sein. Über einen Bedienungsknopf (6) kann eine Transportvorrichtung (5) bedient werden, die beispielsweise über eine Spiralfeder Lanzetten in die Entnahmeposition (33) bringt. Im Inneren der Öffnung (9) des Einführstutzens (37) kann wiederum ein Führungszapfen (29) vorgesehen sein, der in Wechselwirkung zu einer Führungsnut in der Stechhilfe treten kann.

In Figur 6 C und D sind eine Aufsicht sowie eine Ansicht von vorne eines Lanzettensatzes (35) abgebildet, wobei die Ansicht von vorne teilweise angeschnitten ist. Der Lanzettensatz (35) besteht aus einer Vielzahl von Lanzetten (2), die sternförmig um eine kreisförmige, zentrale Kunststoffscheibe (36) angeordnet sind. Dabei stecken die Spitzen der Metallnadeln (11) der Lanzetten (2) in der zentralen Kunststoffscheibe (36). Die Lanzetten selbst entsprechen im wesentlichen den Lanzetten, wie sie in Figur 4 C abgebildet sind. Auch hier sind wieder V-förmige Aussparungen (23) im Lanzettenkörper (12) vorgesehen, die ein Wechselwirken mit dem Lanzettenhalter einer Stechhilfe ermöglichen.

Das Lanzettenmagazin (1) der in den Figuren 6 A und B gezeigten bevorzugten Ausführungsform, kann vom Benutzer geöffnet werden, um jeweils einen neuen Lanzettensatz (35) einzulegen. Das Gehäuse des Lanzettenmagazins (1) kann durch Abnehmen der oberen, d. h. mit einem Sichtfenster versehenen Gehäusehälfte geöffnet werden. Die Lanzetten (2) werden als Lanzettensatz (35) eingelegt. Durch Drehen des eingelegten Lanzettensatzes (35) um seine zentrale Achse wird die Spiralfeder der Transportvorrichtung (5) gespannt. Schließlich wird die abgenommene Gehäusehälfte wieder auf dem Magazin (1) befestigt, beispielsweise durch Verklipsen. Das Magazin (1) ist somit gebrauchsfertig.

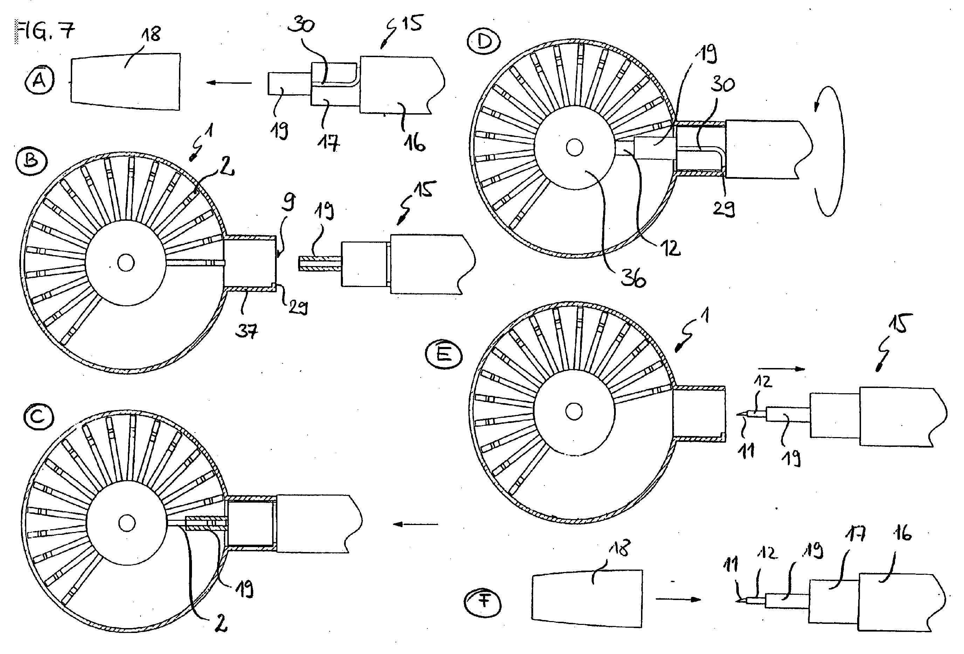

In Figur 7 ist - analog zu Figur 3 und Figur 5 - schematisch anhand von 6 Teilfiguren (A bis F) dargestellt, wie mit einer Stechhilfe (15) Lanzetten (2) aus dem besonders bevorzugten Lanzettenmagazin (1) gemäß Figur 6 entnommen werden können:
Nachdem die Schutzkappe (18) von der Stechhilfe (15) abgenommen wurde, liegen die Spitze (17) mit der Führungsnut (30) sowie der Lanzettenhalter (19) der Stechhilfe (15) frei (Figur 7 A). Die Stechhilfe (15) kann nunmehr in die Öffnung (9) des Einführstutzens (37) des Lanzettenmagazins (1) eingeführt werden. Dabei sorgt die Führungsnut (30) in der Spitze (17) der Stechhilfe (15) im Zusammenspiel mit dem Führungszapfen (29) in der Öffnung (9) des Lanzettenmagazins (1) für eine eindeutige Orientierung der Stechhilfe (15) relativ zum Lanzettenmagazin (1). Sobald die Stechhilfe (15) vollständig mit ihrer Spitze (17) in das Lanzettenmagazin (1) eingeführt ist, wird die Lanzette (2), die sich in der Entnahmeposition befindet, vom Lanzettenhalter (19) gegriffen (Figur 7 B und C).Durch Drehen der Stechhilfe (15), welches durch die Kontur der Führungsnut (30) bewirkt wird, wird die Lanzette (2) zusammen mit der Stechhilfe (15) um ihre Längsachse gedreht. Dabei wird die Spitze der Metallnadel (11) in der Kunststoffscheibe (36) verdreht und somit gelöst (Figur 7 D). Beim Entnehmen der Stechhilfe (15) aus dem Lanzettenmagazin (1) wird die Lanzette (2) vom Lanzettenhalter (19) in der Stechhilfe (15) gehalten (Figur 7 E). Die Spitze der Metallnadel (11) sowie der der Spitze der Metallnadel (11) am nächsten gelegene Teil des Lanezettenkörpers (12) ragen in dieser besonders bevorzugten Ausführungsform aus dem Lanzettenhalter (19) der Stechhilfe (15) heraus. Zum Spannen der Stechhilfe (15) muß wiederum die Schutzkappe (18) über die Spitze (17) mit Lanzettenhalter (19) der Stechhilfe (15) geschoben und relativ zum Griffkörper (16) verdreht werden.

## Patentansprüche

1. System geeignet zur Gewinnung einer Körperflüssigkeit aus einer Körperpartie einer zu untersuchenden Person enthaltend:
- eine längliche, zylindrische Stechhilfe (15), die zur Aufnahme einer Lanzette (2) geeignet ist, einen Griffkörper (16) besitzt, in dessen Inneren ein Großteil eines Mechanismus beherbergt wird, der die Lanzette (2) beim Stechvorgang geführt auf die gewünschte Einstechstelle in der Haut der zu untersuchenden Person zu- und nach dem Stechen wieder wegbewegt, und
einen Lanzettenhalter (19) an einem Ende der Stechhilfe (15), welches als Spitze bezeichnet wird, wobei
der Lanzettenhalter (19) Mittel enthält, die ein Greifen der Lanzette (2) ermöglichen und die somit geeignet sind, eine Lanzette aus dem Lanzettenmagazin zu entnehmen
- ein Lanzettenmagazin (1) zur Bevorratung von zwei oder mehreren Lanzetten (2), wobei das Lanzettenmagazin (1)
ein Gehäuse (3) besitzt, in dem die Lanzetten (2) aufbewahrt werden,
eine Entnahmestelle (4) für eine Lanzette (2) sowie
eine Transportvorrichtung (5) für die Lanzetten (2) aufweist, mit der die Lanzetten (2) manuell oder automatisch in die Entnahmestelle transportiert werden können, und
eine Öffnung (9) besitzt, in welche die Stechhilfe (15) zumindest mit ihrer Spitze zur Entnahme einer einzelnen Lanzette (2) aus dem Innern des Lanzettenmagazins (1) eingeführt werden kann, und
- zwei oder mehrere Lanzetten (2).

2. System gemäß Anspruch 1, **dadurch gekennzeichnet, dass** die Lanzetten nebeneinander, sich paarweise berührend im Lanzettenmagazin angeordnet sind.

3. System gemäß Anspruch 1 oder 2, **dadurch gekennzeichnet, dass** sich im Lanzettenmagazin außer der Öffnung, in welche die Stechhilfe zur Entnahme einer einzelnen Lanzette aus dem Lanzettenmagazin eingeführt werden kann, eine zweite Öffnung befindet, durch welche Schutzhüllen der Lanzettennadeln ausgeworfen werden können.

4. System gemäß einem der Ansprüche 1 bis 3, **dadurch gekennzeichnet, dass** die Transportvorrichtung des Lanzettenmagazins ein manuell zu bedienender Schieber ist.

5. System gemäß einem der Ansprüche 1 bis 3, **dadurch gekennzeichnet, dass** die Transportvorrichtung des Lanzettenmagazins durch einen Federmechanismus betrieben wird.

6. System gemäß einem der Ansprüche 1 bis 5, **dadurch gekennzeichnet, dass** in der Öffnung des Lanzettenmagazins, in welche die Stechhilfe zur Entnahme einer einzelnen Lanzette aus dem Lanzettenmagazin eingeführt werden kann, ein Zapfen vorhanden ist, der in eine Führungsnut in der Spitze der Stechhilfe, welche in die besagte Öffnung eingeführt wird, eingreift und so ein Drehen der Stechhilfe oder eines Teils der Stechhilfe um ihre Längsachse bewirkt.

7. System gemäß einem der Ansprüche 1 bis 5, **dadurch gekennzeichnet, dass** in der Öffnung des Lanzettenmagazins, in welche die Stechhilfe zur Entnahme einer einzelnen Lanzette aus dem Lanzettenmagazin eingeführt werden kann, eine Führungsnut vorhanden ist, in die ein Zapfen in der Spitze der Stechhilfe, welche in die besagte Öffnung eingeführt wird, eingreift und so ein Drehen der Stechhilfe oder eines Teils der Stechhilfe um ihre Längsachse bewirkt.

8. Lanzettenmagazin zur Bevorratung von zwei oder mehreren Lanzetten, welches zur Verwendung in einem System gemäß Anspruch 1 bis 7 geeignet ist, **dadurch gekennzeichnet, dass**
das Lanzettenmagazin
- ein Gehäuse (3) besitzt, in dem die Lanzetten (2) aufbewahrt werden, eine Entnahmestelle (4) für eine Lanzette (2) sowie eine Transportvorrichtung (5) für die Lanzetten (2) aufweist, mit der die Lanzetten (2) manuell oder automatisch in die Entnahmestelle transportiert werden können, und eine Öffnung (9) besitzt, in welche die Stechhilfe (15) zumindest mit ihrer Spitze zur Entnahme einer einzelnen Lanzette (2) aus dem Innern des Lanzettenmagazins (1) eingeführt werden kann

9. Lanzettenmagazin gemäß Anspruch 8, **dadurch gekennzeichnet, dass** sich im Lanzettenmagazin gegenüber der Öffnung, in welche die Stechhilfe zur Entnahme einer Lanzette aus dem Lanzettenmagazin eingeführt werden kann, eine zweite Öffnung befindet, durch welche die Schutzhülle der Lanzettennadel ausgeworfen werden kann.

10. Lanzettenmagazin gemäß Anspruch 8 oder 9, **dadurch gekennzeichnet, dass** in der Öffnung des Lanzettenmagazins, in welche die Stechhilfe zur Entnahme einer einzelnen Lanzette aus dem Lanzettenmagazin eingeführt werden kann, eine Führungsnut oder ein Führungszapfen vorhanden ist.

11. Lanzettenmagazin gemäß einem der Ansprüche 8 bis 10, **dadurch gekennzeichnet, dass** das Lanzettenmagazin Mittel enthält, die es erlauben, den aktuellen Inhalt an Lanzetten von außen zu erkennen.

12. Verwendung des Systems gemäß Anspruch 1 bis 7 in einem Verfahren zur Entnahme einer Lanzette aus einem Lanzettenmagazin, wobei
- eine Lanzette, die sich im Lanzettenmagazin befindet, manuell oder automatisch mit Hilfe einer im Lanzettenmagazin vorhandenen Transportvorrichtung in eine Entnahmeposition im Inneren des Lanzettenmagazins transportiert wird,
- eine Stechhilfe teilweise in eine dafür vorgesehene Öffnung des Lanzettenmagazins eingeführt wird, wobei beim teilweisen Einführen der Stechhilfe diese automatisch die Lanzette, die sich in der Entnahmeposition befindet, greift, und
- die Stechhilfe mit der gegriffenen Lanzette aus dem Lanzettenmagazin herausgenommen wird.

13. Verwendung gemäß Anspruch 12, **dadurch gekennzeichnet, dass** beim Greifen der Lanzette durch die Stechhilfe die Schutzhülle der Lanzettennadel von dieser entfernt wird.

14. Verwendung gemäß Anspruch 12, **dadurch gekennzeichnet, dass** die Schutzhülle der Lanzettennadel beim Herausnehmen der Stechhilfe mit der gegriffenen Lanzette von dieser entfernt wird.

## Claims

1. System that is suitable for collecting a body fluid from a region of the body of a person to be examined comprising:
- an elongate, cylindrical lancing device (15) which is suitable for holding a lancet (2),
has a handle body (16) in the interior of which a large part of a mechanism is accommodated which moves the lancet (2) in a guided manner onto the desired puncture position in the skin of the person to be examined during the lancing process and moves it away again after the lancing, and
a lancet holder (19) at one end of the lancing device (15) which is referred to as a tip wherein the lancet holder (19) contains means which enable the lancet (2) to be gripped and which are thus suitable for removing a lancet from the lancet magazine,
- a lancet magazine (1) for storing two or more lancets (2) wherein the lancet magazine (1) has a housing (3) in which the lancets (2) are stored, a removal point (4) for a lancet (2) and
a transport device (5) for the lancets (2) with which the lancets (2) can be transported manually or automatically into the removal point, and has an opening (9) into which at least the tip of the lancing device (15) can be inserted to remove an individual lancet (2) from the inside of the lancet magazine (1) and
- two or more lancets (2).

2. System according to claim 1, **characterized in that** the lancets are arranged next to one another in pairwise contact in the lancet magazine.

3. System according to claim 1 or 2, **characterized in that** a second opening through which protective sheaths of the lancet needles can be ejected is located in the lancet magazine in addition to the opening into which the lancing device can be inserted to remove a single lancet from the lancet magazine.

4. System according to one of the claims 1 to 3, **characterized in that** the transport device of the lancet magazine is a manually operated slide.

5. System according to one of the claims 1 to 3, **characterized in that** the transport device of the lancet magazine is driven by a spring mechanism.

6. System according to one of the claims 1 to 5, **characterized in that** a pin is present in the opening of the lancet magazine into which the lancing device can be inserted to remove a single lancet from the lancet magazine, and the pin engages in a guide groove in the tip of the lancing device which is inserted into the said opening and thus causes a rotation of the lancing device or of a part of the lancing device about its longitudinal axis.

7. System according to one of the claims 1 to 5, **characterized in that** a guide groove is present in the opening of the lancet magazine into which the lancing device can be inserted to remove a single lancet from the lancet magazine, into which a pin in the tip of the lancing device which is inserted into the said opening engages and thus causes a rotation of the lancing device or of a part of the lancing device about its longitudinal axis.

8. Lancet magazine for storing two or more lancets which is suitable for use in a system according to claims 1 to 7, **characterized in that** the lancet magazine has
- has a housing (3) in which the lancets (2) are stored,
a removal point (4) for a lancet (2) and
a transport device (5) for the lancets (2) with which the lancets (2) can be transported manually or automatically into the removal point, and has an opening (9) into which at least the tip of the lancing device (15) can be inserted to remove an individual lancet (2) from the inside of the lancet magazine (1).

9. Lancet magazine according to claim 8, **characterized in that** a second opening through which the protective sheath of the lancet needle can be ejected is located in the lancet magazine opposite to the opening into which the lancing device can be inserted to remove a lancet from the lancet magazine.

10. Lancet magazine according to claim 8 or 9, **characterized in that** a guide groove or a guide pin is present in the opening of the lancet magazine into which the lancing device can be inserted to remove a single lancet from the lancet magazine.

11. Lancet magazine according to one of the claims 8 to 10, **characterized in that** the lancet magazine contains means which allow the actual content of lancets to be identified from outside.

12. Use of the system according to claims 1 to 7 in a method for removing a lancet from a lancet magazine, wherein
- a lancet located in the lancet magazine is manually or automatically transported into a removal position in the interior of the lancet magazine with the aid of a transport device present in the lancet magazine
- a lancing device is partially inserted into an opening provided for this purpose in the lancet magazine, wherein the lancing device when partially inserted automatically grips the lancet located in the removal position and
- the lancing device with the gripped lancet is removed from the lancet magazine.

13. Use according to claim 12, **characterized in that** the protective sheath of the lancet needle is removed from it when the lancing device grips the lancet.

14. Use according to claim 12, **characterized in that** the protective sheath of the lancet needle is removed from it when the lancing device with the gripped lancet is removed.

## Revendications

1. Système approprié pour l'extraction d'un fluide corporel d'une partie du corps d'une personne à examiner, comprenant :
- un autopiqueur (15) oblong cylindrique qui est approprié pour
la réception d'une lancette (2),
qui possède un corps de poignée (16),
à l'intérieur duquel est logé une grande partie d'un mécanisme qui déplace la lancette (2) lors du processus de piqûre de façon guidée vers le point de piqûre souhaité sur la peau de la personne à examiner et l'en retire après la piqûre, et
un support de lancettes (19) à une extrémité de l'autopiqueur (15) qui est définie en pointe, dans lequel
le support de lancettes (19) comprend des moyens qui permettent une préhension de la lancette (2) et sont ce faisant appropriés pour extraire une lancette du distributeur de lancettes
- un distributeur de lancettes (1) pour l'approvisionnement de deux ou plusieurs lancettes (2), dans lequel le distributeur de lancettes (1)
possède un boîtier (3) dans lequel les lancettes (2) sont conservées, présente un point de prélèvement (4) pour une lancette (2) ainsi qu'un dispositif de transport (5) pour les lancettes (2)
avec lequel les lancettes (2) peuvent être transportées manuellement ou automatiquement dans le point de prélèvement, et
possède une ouverture (9) dans laquelle l'autopiqueur (15) peut au moins être introduit avec sa pointe pour le prélèvement d'une lancette (2) unique de l'intérieur du distributeur de lancettes (1), et
- deux ou plusieurs lancettes (2).

2. Système selon la revendication 1, **caractérisé en ce que** les lancettes sont disposées les unes à côté des autres et de manière contiguë par paires dans le distributeur de lancettes.

3. Système selon la revendication 1 ou 2, **caractérisé en ce que,** outre l'ouverture dans laquelle l'autopiqueur peut être introduit pour le prélèvement d'une lancette unique dans le distributeur de lancettes, se trouve une seconde ouverture dans le distributeur de lancettes, via laquelle des gaines protectrices des aiguilles de lancettes peuvent être éjectées.

4. Système selon l'une des revendications 1 à 3, **caractérisé en ce que** le dispositif de transport du distributeur de lancettes est un curseur à actionner manuellement.

5. Système selon l'une des revendications 1 à 3, **caractérisé en ce que** le dispositif de transport du distributeur de lancettes est actionné par un mécanisme à ressort.

6. Système selon l'une des revendications 1 à 5, **caractérisé en ce que**, dans l'ouverture du distributeur de lancettes dans laquelle l'autopiqueur peut être introduit pour le prélèvement d'une lancette unique dans le distributeur de lancettes, se trouve un tourillon qui s'engrène dans une rainure d'introduction dans la pointe de l'autopiqueur, lequel est introduit dans ladite ouverture, et provoque ce faisant une rotation de l'autopiqueur ou d'une partie de l'autopiqueur le long de son axe longitudinal.

7. Système selon l'une des revendications 1 à 5, **caractérisé en ce que**, dans l'ouverture du distributeur de lancettes dans laquelle l'autopiqueur peut être introduit pour le prélèvement d'une lancette unique dans le distributeur de lancettes, se trouve une rainure d'introduction dans laquelle s'engrène un tourillon dans la pointe de l'autopiqueur, lequel est introduit dans ladite ouverture, et provoque ce faisant une rotation de l'autopiqueur ou d'une partie de l'autopiqueur le long de son axe longitudinal.

8. Distributeur de lancettes pour l'approvisionnement de deux ou plusieurs lancettes, lequel est approprié pour une utilisation dans un système selon la revendication 1 à 7, **caractérisé en ce que**
le distributeur de lancettes
- possède un boîtier (3) dans lequel les lancettes (2) sont conservées, présente un point de prélèvement (4) pour une lancette (2) ainsi qu'un dispositif de transport (5) pour les lancettes (2)
avec lequel les lancettes (2) peuvent être transportées manuellement ou automatiquement dans le point de prélèvement, et
possède une ouverture (9) dans laquelle l'autopiqueur (15) peut au moins être introduit avec sa pointe pour le prélèvement d'une lancette (2) unique de l'intérieur du distributeur de lancettes (1).

9. Distributeur de lancettes selon la revendication 8, **caractérisé en ce que,** face à l'ouverture dans laquelle l'autopiqueur peut être introduit pour le prélèvement d'une lancette dans le distributeur de lancettes, se trouve une seconde ouverture dans le distributeur de lancettes, via laquelle la gaine protectrice de l'aiguille de lancette peut être éjectée.

10. Distributeur de lancettes selon la revendication 8 ou 9, **caractérisé en ce que**, dans l'ouverture du distributeur de lancettes dans laquelle l'autopiqueur peut être introduit pour le prélèvement d'une lancette unique dans le distributeur de lancettes, se trouve un tourillon d'introduction ou une rainure d'introduction.

11. Distributeur de lancettes selon l'une des revendications 8 à 10, **caractérisé en ce que** le distributeur de lancettes comprend des moyens qui permettent de reconnaître le contenu réel de lancettes de l'extérieur.

12. Utilisation du système selon la revendication 1 à 7 dans un procédé de prélèvement d'une lancette dans un distributeur de lancettes, dans laquelle une lancette qui se trouve dans le distributeur de lancettes est transportée manuellement ou automatiquement à l'aide d'un dispositif de transport disponible dans le distributeur de lancettes dans une position de prélèvement à l'intérieur du distributeur de lancettes, un autopiqueur est partiellement introduit dans une ouverture du distributeur de lancettes prévue à cet effet, dans laquelle lors de l'introduction partielle de l'autopiqueur celui-ci saisit automatiquement la lancette qui se trouve en position de prélèvement et l'autopiqueur est extrait du distributeur de lancettes avec la lancette saisie.

13. Utilisation selon la revendication 12, **caractérisée en ce que**, lors de la saisie de la lancette par l'autopiqueur, la gaine protectrice de l'aiguille de lancette est ôtée de celle-ci.

14. Utilisation selon la revendication 12, **caractérisée en ce que** la gaine protectrice de l'aiguille de lancette est ôtée de celle-ci lors de l'extraction de l'autopiqueur avec la lancette saisie.
